# EUROPEAN PATENT SPECIFICATION

(11) **EP 1 509 131 B1**
(45) Date of publication and mention of the grant of the patent: **17.09.2008**
(21) Application number: 03752960.9
(22) Date of filing: 14.05.2003
(51) Int. Cl.: A61B 5/145

(54) **SENSOR UNIT AND METHOD FOR SENSING A BLOOD RELATED PARAMETER AND SYSTEM INCLUDING SUCH A SENSOR UNIT**
MESSFÜHLEREINHEIT UND VERFAHREN ZUR MESSUNG EINES BLUTRELEVANTEN PARAMETERS UND SYSTEM MIT EINER SOLCHEN MESSFÜHLEREINHEIT
CAPTEUR ET PROCEDE DE DETECTION D'UN PARAMETRE ASSOCIE AU SANG ET SYSTEME COMPRENANT UN TEL CAPTEUR

(30) Priority: 17.05.2002 SE 0201497
(43) Date of publication of application: 02.03.2005
(73) Proprietor: HEMAPURE AB, 751 09 Uppsala (SE)
(72) Inventor: DANIELSON, Bo, G., S-752 46 Uppsala (SE); PERSSON, Dick, S-820 50 Los (SE)
(74) Representative: Janson, Ronny
(86) International application number: PCT/SE2003/000784
(87) International publication number: WO 2003/096903

(56) References cited:
- EP-A1- 0 354 736
- EP-A1- 1 048 264
- US-A- 5 243 982
- US-A- 6 090 048
- US-B1- 6 208 880

## Description

### Field of the invention

The invention concerns a sensor unit for sensing a blood related parameter in a bloodstream of a human or an animal. It also concerns a method for monitoring the function of a bloodstream access device and a blood parameter sensor system including such a sensor unit.

### Description of Prior Art

Various devices are previously known for sensing various parameters in the bloodstream of a human. The most common arrangement for measuring such parameters is by introducing a catheter including a sensor element into body tissue of the patient. This procedure however is somewhat problematic, since the sensor element may be positioned outside an intended blood vessel which can result in erroneous measurements. Another drawback is that it is often necessary to punch the patient's skin at regular intervals in order to gain proper access to the patient's bloodstream, since this solution is not suitable for permanent use.

US-A-5 120 313 discloses a method for measuring blood pressure in an animal or human using a percutaneous access port. This access port includes a base assembly which is intended to be implanted into the animal or human and comprises an inlet port which is adapted to be connected to a selected organ in order to provide communication with a fluid chamber inside the base assembly. A transducer inside the fluid chamber of the base assembly is adapted to detect the pressure of the fluid so as to provide a signal indicative of the blood pressure of the human or the animal.

The described arrangement is equipped with a pump for continuously flushing the system including a catheter for connection to the bloodstream of the human or the animal in order to prevent formation of blood clots. Altogether the described arrangement is an insecure, complicated, limited and expensive solution which appears to be mainly intended for use in animals in connection with medical research.

It is also referred to US-B1-6208880, which concerns a blood parameter measurement device and to EP-A1-1048264, which concerns a closed loop medicament pump.

### Aim and most important features of the invention

It is an aim of this invention to provide a solution to the prior art problems and in particular to provide a secure, inexpensive and easy handled sensor arrangement.

This aim is achieved in a sensor unit as above according to the features of the characterising portion of claim 1.

The inventive sensor unit provides for easy application in or on a bloodstream access device and guarantees that the sensor element is operative with respect of a continuous blood flow, thus guaranteeing that sensing of the blood related parameter is carried out accurately.

It is an advantage to use a bloodstream access device which preferably is implanted for permanent use. Normally such devices are implanted so as to allow blood treatment such as blood faltering in case of patients with a renal failure diagnosis. According to this aspect of the invention such a device is used as part of the sensor system besides its normal use for blood treatment.

One example of a preferred bloodstream access device is previously known from WO 99/20338 (Hemapure AB). It is preferred that a lid member being included in the sensor unit of the present invention is adapted to be applied to a bloodstream access device according to that document in a manner which is thoroughly described therein.

Allowing a continuous blood flow during operation of the sensor element avoids formation of clots etc. and contributes to the sensor unit according to the invention being a long life solution.

An inventive method, as defined in claim 6, provides for monitoring the function of the bloodstream access device. Hereby the blood temperature is sensed in a channel which allows a continuous blood flow passing between an artery side and a vein side of the bloodstream access device. The sensed value is used as an indication of the blood flow through the bloodstream access device. Low flow and low temperature might indicate malfunction of the access device for various reasons and should at least trigger a control of the device by skilled personnel.

Further advantages are achieved with respect to further aspects of the invention and will be explained in the following detailed description.

### Brief description of drawings

The invention will now be described in more detail at the background of preferred embodiments and with reference to the drawings, wherein:
Fig. la shows a bloodstream access device for use in connection with a system according to the invention,
Figs. 2a and 2b show an arrangement including a bloodstream access device with attached sensor unit according to the invention,
Fig. 3a and 3b show an alternative sensor unit, and
Fig. 4 shows a system including an inventive sensor unit in respect of a human.

### Description of embodiments

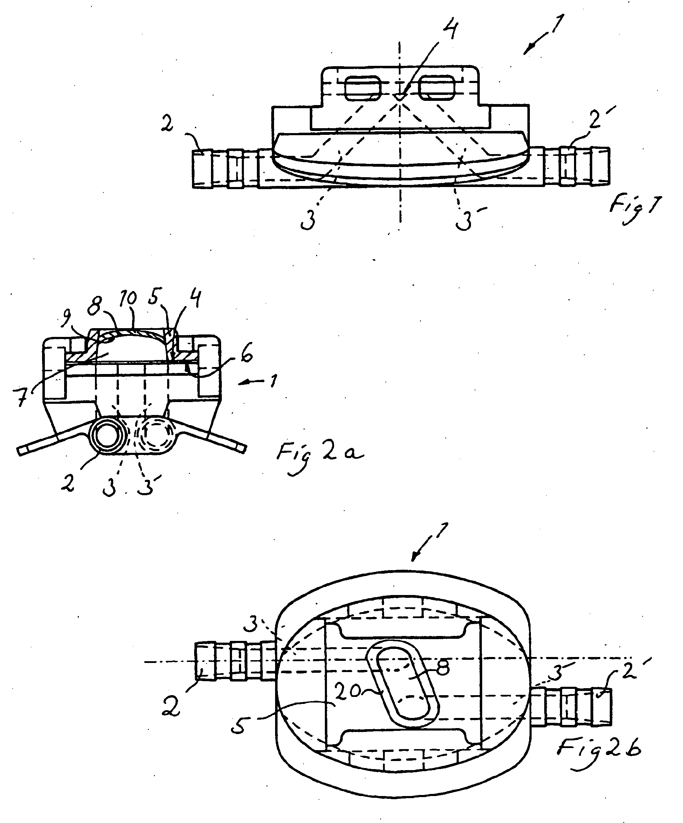

In Fig. 1 a bloodstream access device 1 is shown having nipples 2 and 2' for connection to an artery and a vein of a human or an animal. Channels 3 and 3' interconnect the nipples with a flat or curved interface surface 4 which is intended for connection to a connection device belonging to an external circuit. Such a circuit could include an artificial kidney. The interface is co-operating with a lid between blood treatments, said lid including a channel for allowing a continuous flow between the artery and the vein side through the channels 3 and 3'.

Fixing the different lids and connection devices onto the bloodstream access device is not part of the invention, but for understanding reference is made to the above mentioned document WO 99/20338. The device 1 is intended for permanent implantation into a human or an animal and includes means for allowing ingrowth of body tissue for stabilising purposes.

In Fig. 2a the bloodstream access device 1 in Fig. 1 is shown in a view from the right in Fig. 1 carrying a sensor unit 5 which is shown in a section. The sensor unit 5 co-operates with the bloodstream access device to that extent that it provides sealing co-operation between a sealing surface 6 and the surface of the interface 4 of the bloodstream access device 1. The sensor unit 5 is thus positioned and fixed on the bloodstream access device in a manner which is described in the above-mentioned WO document.

Further, the sensor unit 5 has a channel portion 7 which is intended to interconnect the channels 3 and 3' of the bloodstream access device 1 so as to allow a continuous blood flow through the device with the applied sensor unit 5. At the top the channel 7 is limited by a wall including a sensor element 8 comprising a sensor layer 9 which comprises the inner wall portion of that part of the channel portion 7.

Further, the sensor element 8 includes a translucent protective layer 10 which protects the sensor layer 9 from being affected from outside mechanical influences as well as, in certain applications, provides a certain thermal insulation so as not to cool down the blood streaming through the channel 7.

The sensor layer 9 includes preferably liquid crystals which are sensitive to the parameter which at present is intended to be sensed. It may thus for example be temperature sensitive or sensitive to a defined substance in the bloodstream such as insulin or glucose. As is per se known, the sensor layer may be of a kind which changes colour so as provide a visual indication of the parameter level, be it temperature, concentration etc., of the blood being present inside the channel 7.

Temperature indication may in turn be used as a measurement of the flow through the channel 7. I.e. in principle, high flow results in high temperature; low flow results in lower temperature. Flow indication in turn may be used for monitoring the function of the bloodstream access device and absence of clots etc. However, temperature indication could also be used for monitoring the certain ill-health conditions of the patient. An ideal temperature might be for example 35°C and higher or lower temperatures might indicate some kind of problem.

Taken more generally, the indication could be used as a warning or as a trigger for introducing medication into the patient.

In case the sensor layer includes sensor elements that are indicative of the concentration of a specific substance in the bloodstream, this may be indicated similarly, that is with liquid crystals which have the capability to change colour or in any other per se known manner.

Fig. 2b shows the bloodstream access device 1 from above with a fastened sensor unit 5. The sensor element 8 is shown as an oblong element in the centre of the sensor unit 5. In case of liquid crystal material having the provision to change colour as a response to sensed parameter values, it is preferred that the sensor element 8 is surrounded by a surface 20 having a colour scale. Hereby easy comparison with the present colour of the sensor element 8 is enabled. This makes it possible to have a quick indication on the temperature and thus the function of the device 1 or, at occasions, the concentration level etc. prevailing inside channel 7.

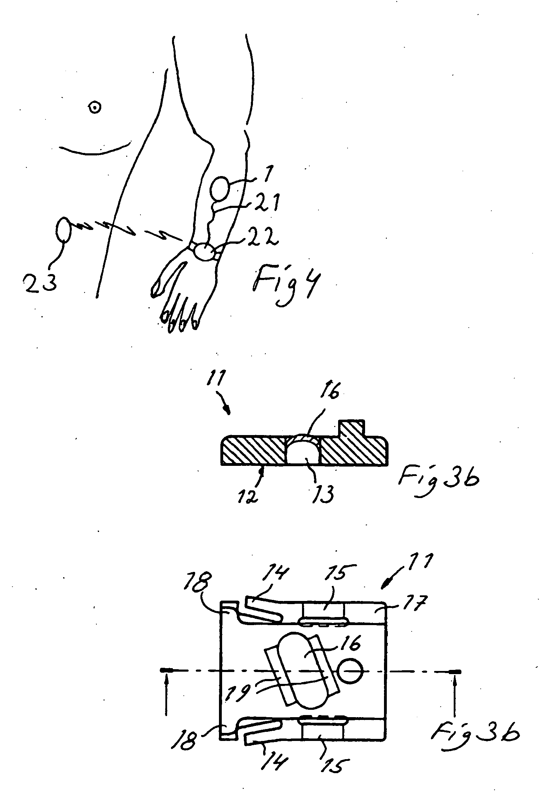

A second embodiment of a sensor unit 11 is shown in fig 3a. In accordance with connecting parts described in the above-mentioned WO document, the sensor unit is provided with snap wings 14, and pressing portions 15 on holding means 17. Further, locking elements or shoulders 18 are used for positioning the sensor unit 11 on a bloodstream access device 1 (Fig. 1).

An oblong sensor element 16 is positioned centrally and with an acute angle with respect to a longitudinal axis of the sensor unit 11. At the sides of the sensor element 16, there are indicated two areas 19 which may be coloured in scales so as to provide easy indication of flow, temperature, concentration etc. etc.

Fig. 3b shows the sensor unit 11 in a longitudinal section wherein the curved sensor element 16 contributes to limit a channel 13, which corresponds to the channel 7 of the sensor unit 5 in Fig. 2a.

The invention may be modified further and for example the sensor units 5 and 11, which in practice are lid members for co-operation with the bloodstream access device, may be connected to outside equipment for indicating sensed temperature, flow concentration etc. This is indicated in fig 4, which shows a system including an inventive sensor unit in respect of a human. In this case the lid member may be connected, for example over an electric wire 21 or wireless, with a wrist carried indicator 22 resembling a wrist-watch.

It is also possible to combine the sensor unit with other kinds of equipment. For example in case of the sensor unit includes a sensor element for sensing blood glucose level, an inventive sensor unit may be connected to an insulin pump 23 for introducing insulin into the body of the patient. A simple external device may be a warning indicator such as a buzzer, a beeper or a lamp, indicating malfunction or parameter levels below a predetermined value.

As examples, blood temperature, blood flow, blood-pressure, level of biochemical substances such as glucose, insulin, urea may be subject to being sensed by adapted sensor elements of a sensor unit according to the invention.

Further modifications may include other per se known sensor elements; use in connection with other types of bloodstream access devices; and connections to other kinds of indicators and external equipment.

## Claims

1. Sensor unit (5;11) including at least one sensor element (8;16) for sensing a blood related parameter in a bloodstream of a human or an animal, said sensor unit being connectable to a main body of a bloodstream receiving device (1) which includes connecting elements (2,2') for connection to an artery and a vein of the human or the animal, wherein the sensor unit comprises a channel (7;13) for allowing a continuous blood flow passing between an artery side and a vein side of the bloodstream receiving device (1), and wherein the sensor element(s) (8;16) is (are) positioned for sensing the blood related parameter(s) in the channel (7;13),
**characterized in**
- **that** the sensor unit (5;11) includes a lid member for connection to the bloodstream receiving device, which is in the form of a bloodstream access device (1) having an interface surface (4) which is intended for connection to a connection device belonging to an external circuit,
- **that** the sensor unit (5) is arranged with a sealing surface (6) so that it can co-operate with the bloodstream access device to the extent that it provides sealing co-operation between the sealing surface (6) and the surface of the interface (4) of the bloodstream access device (1).

2. Sensor unit according to claim 1, **characterized in that** it includes a parameter level indicator.

3. Sensor unit according to claim 1, **characterized in that** it includes means (21) for transmitting parameter related signals to an external receiver (22).

4. Sensor unit according to claim 1, 2 or 3, **characterized in that** the sensor element (8;16) is a liquid crystal sensor.

5. Sensor unit according to any one of the claims 1 - 4, **characterized in that** the sensor element (8;16) is a sensor for one parameter in the group: blood temperature, blood flow, blood-pressure, level of biochemical substances such as glucose, insulin, urea.

6. Method for monitoring the function of a bloodstream access device, including the steps:
- connecting a lid member, being included in a sensor unit (5; 11), and having a channel (7; 13), to a main body of a bloodstream access device (1) having an interface surface (4) which is intended for connection to a connection device belonging to an external circuit which channel allows a continuous blood flow passing between an artery side and a vein side of the bloodstream access device (1), said sensor unit being arranged with a sealing surface (6) so that it can co-operate with the bloodstream access device to the extent that it provides sealing cb-operation between the sealing surface (6) and the surface of the interface (4) of the bloodstream access device (1),
- sensing blood temperature in the channel (7;13) of said lid member, and
- using the sensed value as an indication of the blood flow through the bloodstream access device.

7. Blood parameter sensor system, **characterized in that** it includes a bloodstream access device (1) and a sensor unit (5;11) according to any of the claims 1 - 5.

8. System according to claim 7, **characterized in that** it includes a feed-back device (23) for administration of a substance into the human or the animal as a response to a sensed parameter level.

9. System according to claim 8, **characterized in that** the feed-back device (23) includes a controlled pump for the substance.

## Patentansprüche

1. Sensoreinheit (5; 11) enthaltend mindestens ein Sensorelement (8; 16) zum Erkennen eines blutbezogenen Parametern in einem Blutstrom eines Menschen oder eines Tieres, die Sensoreinheit ist mit einem Hauptkörper einer Blutstromempfängervorrichtung (1), welche Verbindungselemente (2, 2') zur Verbindung mit einer Arterie und einer Vene des Menschen oder des Tieres enthält, verbindbar, wobei die Sensoreinheit einen Kanal (7; 13) zum Ermöglichen eines kontinuierlichen Blutstroms zwischen einer Arterienseite und einer Venenseite der Blutstromempfängervorrichtung (1) umfasst, und wobei das Sensorelement/die Sensorelemente (8; 16) zum Erkennen des oder der blutbezogenen Parameter (in dem Kanal (7; 13) angeordnet ist/sind,
**dadurch gekennzeichnet,**
- **dass** die Sensoreinheit (5; 11) ein Deckelelement zur Verbindung mit der Blutstromempfängervorrichtung enthält, welche in Form einer Blutstromzugangsvorrichtung (1) mit einer Schnittstellenfläche (4), die zur Verbindung mit einer zu einem externen Kreislauf gehörenden Verbindungsvorrichtung vorgesehen ist, ausgebildet ist
- **dass** die Sensoreinheit (5) mit einer Dichtfläche (6) ausgestattet ist, so dass sie mit der Blutstromzugangsvorrichtung insofern zusammenwirken kann, als dass sie eine dichtende Zusammenwirkung zwischen der Dichtfläche (6) und der Fläche der Schnittstelle (4) der Blutstromzugangsvorrichtung (1) bereitstellt.

2. Sensoreinheit nach Anspruch 1, **dadurch gekennzeichnet, dass** sie eine Parameterniveauanzeige enthält.

3. Sensoreinheit nach Anspruch 1, **dadurch gekennzeichnet, dass** sie Mittel (21) zum Übertragen von parameterbezogenen Signalen an einen externen Empfänger (22) enthält.

4. Sensoreinheit nach Anspruch 1, 2 oder 3, **dadurch gekennzeichnet, dass** das Sensorelement (8; 16) ein Flüssigkristallsensor ist.

5. Sensoreinheit nach einem der Ansprüche 1 bis 4, **dadurch gekennzeichnet, dass** das Sensorelement (8; 16) ein Sensor für einen Parameter aus der Gruppe von Bluttemperatur, Blutstrom, Blutdruck, Niveau von biochemischen Substanzen wie Glucose, Insulin, Harnstoff ist.

6. Verfahren zum Überwachen der Funktion einer Blutstromzugangsvorrichtung, umfassend die Schritte:
- Verbinden eines in einer Sensoreinheit (5; 11) enthaltenen und einen Kanal (7; 13) aufweisenden Deckelelements mit einem Hauptkörper einer Blutstromzugangsvorrichtung (1), welche eine zur Verbindung mit einer zu einem externen Kreislauf gehörigen Verbindungsvorrichtung vorgesehene Schnittstellenfläche (4) hat, in der ein Kanal einen kontinuierlichen Strom zwischen einer Arterienseite und einer Venenseite der Blutstromzugangsvorrichtung (1) ermöglicht, die Sensoreinheit ist mit einer Dichtfläche (6) ausgestattet, so dass sie mit der Blutstromzugangsvorrichtung insoweit zusammenwirken kann, als dass sie eine dichtende Zusammenwirkung zwischen der Dichtfläche (6) und der Fläche der Schnittstelle (4) der Blutstromzugangsvorrichtung (1) bereitstellt,
- Erkennen einer Bluttemperatur in dem Kanal (7; 13) des Deckelements, und
- Verwenden der erkannten Werte als eine Anzeige für den Blutstrom durch die Blutstromzugangsvorrichtung.

7. Blutparametererkennungssystem, **dadurch gekennzeichnet, dass** es eine Blutstromzugangsvorrichtung (1) und eine Sensoreinheit (5; 11) entsprechend einem der Ansprüche 1 bis 5 enthält.

8. System nach Anspruch 7, **dadurch gekennzeichnet, dass** es eine Rückführvorrichtung (23) zur Zuführung einer Substanz in den Menschen oder das Tier als eine Reaktion auf ein erkanntes Parameterniveau enthält.

9. System nach Anspruch 8, **dadurch gekennzeichnet, dass** die Rückführvorrichtung (23) eine gesteuerte/geregelte Pumpe für die Substanz enthält.

## Revendications

1. Unité de capteur (5 ; 11) comprenant au moins un élément de capteur (8 ; 16) destiné à détecter un paramètre associé au sang dans la circulation sanguine d'un humain ou d'un animal, ladite unité de capteur pouvant être connectée à un corps principal d'un dispositif de réception de circulation sanguine (1) qui comprend des éléments de connexion (2, 2') pour la connexion à une artère et à une veine de l'être humain ou de l'animal, dans lequel l'unité de capteur comprend un canal (7 ; 13) destiné à permettre le passage d'un flux sanguin continu entre un côté artériel et un côté veineux du dispositif de réception de circulation sanguine (1), et dans lequel l'élément (les éléments) de capteur (8 ; 16) est (sont) positionné(s) pour détecter le (les) paramètres associé(s) au sang dans le canal (7 ; 13),
**caractérisé en ce que**
- l'unité de capteur (5 ; 11) comprend un élément de couvercle pour la connexion au dispositif de réception de circulation sanguine, qui se trouve sous la forme d'un dispositif d'accès à la circulation sanguine (1) ayant une surface d'interface (4) qui est destinée à être connectée à un dispositif de connexion appartenant à un circuit externe,
- l'unité de capteur (5) est arrangée avec une surface de scellement (6) de manière à ce qu'elle puisse coopérer avec le dispositif d'accès à la circulation sanguine au point qu'elle fournit une coopération de scellement entre la surface de scellement (6) et la surface de l'interface (4) du dispositif d'accès à la circulation sanguine (1).

2. Unité de capteur selon la revendication 1, **caractérisée en ce qu'**elle comprend un indicateur de niveau de paramètre.

3. Unité de capteur selon la revendication 1, **caractérisée en ce qu'**elle comprend des moyens (21) de transmission de signaux apparentés à un paramètre à un récepteur externe (22).

4. Unité de capteur selon la revendication 1, 2 ou 3, **caractérisée en ce que** l'élément de capteur (8 ; 16) est un capteur à cristaux liquides.

5. Unité de capteur selon l'une quelconque des revendications 1 à 4, **caractérisée en ce que** l'élément de capteur (8 ; 16) est un capteur pour un paramètre dans le groupe : température du sang, débit sanguin, pression sanguine, niveau de substances biochimiques telles que le glucose, l'insuline, l'urée.

6. Procédé de surveillance du fonctionnement d'un dispositif d'accès à la circulation sanguine, qui comprend les étapes consistant à _{:}
- connecter un élément de couvercle, étant inclus dans une unité de capteur (5 ; 11) comprenant un canal (7, 13), à un corps principal d'un dispositif d'accès à la circulation sanguine (1) ayant une surface d'interface (4) qui est destinée à être connectée à un dispositif de connexion appartenant à un circuit externe dont le canal permet le passage d'un flux sanguin continu entre un côté artériel et un côté veineux du dispositif d'accès à la circulation sanguine (1), ladite l'unité de capteur étant arrangée avec une surface de scellement (6) de manière à ce qu'elle puisse coopérer avec le dispositif d'accès à la circulation sanguine au point qu'elle fournit une coopération de scellement entre la surface de scellement (6) et la surface de l'interface (4) du dispositif d'accès à la circulation sanguine (1),
- détecter la température du sang dans le canal (7 ; 13) dudit élément de couvercle, et
- utiliser la valeur détectée comme une indication du débit sanguin dans le dispositif d'accès à la circulation sanguine.

7. Système de capteur de paramètres sanguins, **caractérisé en ce qu'**il comprend un dispositif d'accès à la circulation sanguine (1) et une unité de capteur (5 ; 11) selon l'une quelconque des revendications 1 à 5.

8. Système selon la revendication 7, **caractérisé en ce qu'**il comprend un dispositif à rétroaction (23) pour l'administration d'une substance dans l'être humain ou l'animal en réponse à un niveau de paramètre détecté.

9. Système selon la revendication 8, **caractérisé en ce que** le dispositif à rétroaction (23) comprend une pompe régulée pour la substance.
